# EUROPÄISCHE PATENTSCHRIFT

(11) **EP 1 273 603 B1**
(45) Veröffentlichungstag und Bekanntmachung des Hinweises auf die Patenterteilung: **26.09.2007**
(21) Anmeldenummer: 02010931.0
(22) Anmeldetag: 16.05.2002
(51) Int. Cl.: C08G 18/02, C08G 18/20, C07D 251/34

(54) **Verfahren zur Herstellung von geruchsarmen und lagerstabilen monomerhaltigen Polyisocyanuraten auf Basis von Isophorondiisocyanat**
Process for the preparation of low-odour and storage-stable monomer-containing polyisocyanurates from isophorone diisocyanate
Procédé pour la préparation de polyisocyanurates avec une faible odeur, stables au stockage et contenant des monomères à partir de diisocyanate d'isophorone

(30) Priorität: 02.07.2001 DE 10131525
(43) Veröffentlichungstag der Anmeldung: 08.01.2003
(73) Patentinhaber: Degussa GmbH, 40474 Düsseldorf (DE)
(72) Erfinder: Kohlstruk, Stephan, Dr., 48249 Dülmen (DE); Kreczinski, Manfred, 44652 Herne (DE); Lomölder, Rainer, Dr., 48153 Münster (DE)

(56) Entgegenhaltungen:
- EP-A- 0 003 765
- EP-A- 0 017 998
- EP-A- 1 170 283
- WO-A-99/36455
- DE-A- 2 631 733
- US-A- 4 540 781

## Beschreibung

Die vorliegende Erfindung betrifft ein Verfahren zur Herstellung von geruchsarmen und lagerstabilen monomerhaltigen Polyisocyanuraten auf Basis von Isophorondiisocyanat.

Polyisocyanurate sind als Polyisocyanataddukte wertvolle Komponenten zur Herstellung von hochwertigen Beschichtungen mit guten mechanischen Eigenschaften sowie guter Licht- und Wetterbeständigkeit. Polyisocyanurate auf Basis von Isophorondiisocyanat (IPDI) finden zudem Einsatz als Rohstoff für PUR-basierende Elastomeranwendungen. Dabei kann es wünschenswert sein, dass das IPDI-basierende Polyisocyanurat, man spricht auch von IPDI-Trimer, in monomerhaltiger Form eingesetzt wird.

Grundsätzlich werden Polyisocyanurate durch katalytische Trimersierung geeigneter Isocyanate erhalten. Geeignete Isocyanate sind z. B. aromatische, cycloaliphatische und aliphatische di- und höherwertige Polyisocyanate. Als Katalysatoren kommen z. B. tert. Amine (US 3,996,223), Alkalimetallsalze von Carbonsäuren (CA 2113890; EP 56159), quart. Ammoniumsalze (EP 798299; EP 524501; US 4,186,255; US 5,258,482; US 4,503,226; US 5,221,743), Aminosilane (EP 197864; US 4,697,014) und quart. Hydroxylalkylammoniumsalze (EP 17998; US 4,324,879) in Frage. In Abhängigkeit vom Katalysator ist auch die Verwendung von diversen Co-Katalysatoren möglich, z. B. OH-funktionalisierte Verbindungen oder Mannich Basen aus sek. Aminen und Aldehyden bzw. Ketonen.

DE-A-2631733 beschreibt ein Verfahren zur Förderung von Kondensations- und/oder Polymerisationsreaktionen von organischen Isocyanaten bei der Herstellung von Urethan- bzw. Isocyanatgruppen aufweisenden Produkten unter Verwendung quartärer Hydroxyalkylammoniumverbindungen.

Zur Trimerisierung läßt man die Polyisocyanate in Gegenwart des Katalysators, ggf. unter Verwendung von Lösemitteln und/oder Hilfsstoffen, bis zum Erreichen des gewünschten Umsatzes reagieren. Man spricht in diesem Zusammenhang auch von partieller Trimerisierung, da der angestrebte Umsatz in der Regel deutlich unterhalb von 100 % liegt. Danach wird die Reaktion durch Deaktivierung des Katalysators abgebrochen. Dies erfolgt durch Zusatz eines Katalysatorinhibitors wie beispielsweise p-Toluolsulfonsäure, Chlorwasserstoff oder Dibutylphosphat und hat zwangsläufig eine ggf. unerwünschte Kontamination des entstandenen isocyanuratgruppenhaltigen Polyisocyanats zur Folge.

Besonders vorteilhaft im Hinblick auf die Trimerisierung von Isocyanaten im technischen Maßstab ist der Einsatz von quart. Hydroxyalkylammoniumcarboxylaten als Oligomerisierungskatalysatoren. Dieser Katalysatortyp ist thermisch labil und erlauben eine gezielte thermische Deaktivierung, so dass es unnötig ist, die Trimerisierung bei Erreichen des gewünschten Umsatzes durch Zudosierung potentiell qualitätsmindernder Inhibitoren abzustoppen.

Monomerhaltiges IPDI-Trimer, welches beispielsweise für o. g. PUR-Spritzapplikationen geeignet ist, weist einen NCO-Gehalt von 28 - 32 Gew.-% auf. Das Polyisocyanurat wird durch partielle Trimerisierung von IPDI in Gegenwart eines oder mehrerer geeigneter Katalysatoren hergestellt. Danach muß der Katalysator aus der Reaktionslösung entweder vollständig entfernt - dies kann durch Kurzweg- oder Dünnschichtdestillation erfolgen - oder deaktiviert werden, weil das Trimerisat in Gegenwart aktiver Katalysatorrückstande nicht lagerstabil ist. Liegt der NCO-Gehalt des erhaltenen IPDI-Polyisocyanurats unterhalb des gewünschten Niveaus, kann er durch Verdünnung der Lösung mit monomerem IPDI problemlos wunschgemäß eingestellt werden.

Alkalimetallsalze von Carbonsäuren sind als Katalysatoren für die Herstellung von monomerhaltigem IPDI-Trimer nicht gut geeignet, da sie sich nur schwierig oder gar nicht aus dem Reaktionsprodukt entfernen lassen. In bezug auf die verfügbaren aminhaltigen Katalysatoren hat es sich erwiesen, dass die resultierenden IPDI-Trimer-Lösungen grundsätzlich mit einem deutlich wahrnehmbaren Geruch behaftet sind, der ausgeprägt genug ist, auch in der Endapplikation noch spürbar und unangenehm in Erscheinung zu treten. Zur Beseitigung der Geruchsbelästigung wird die Reaktionslösung in der technischen Praxis nach partieller Trimerisierung und Katalysatordeaktivierung vom überschüssigen IPDI, von geruchsgebenden Komponenten und ggf. von unerwünschten Katalysatoreninhibitoren befreit. Dies geschieht in der Regel durch Kurzweg- oder Dünnschichtdestillation. Im Anschluß wird das monomerbefreite Festharz unter Zudosierung von frischem IPDI in das gewünschte, geruchsarme und monomerhaltige IPDI-Polyisocyanurat überführt.

Die Sequenz aus partieller Trimerisierung/Deaktivierung, Entmonomerisierung/ Reinigung und abschließender Lösung des Festharzes im Monomer ist sehr aufwendig. Zeit- und kostentreibender Schritt und zudem kapazitätslimitierender Engpaß des bestehenden Verfahrens ist vor allem der Schritt der Monomerabtrennung. Ein wirtschaftlicheres Verfahren zur Herstellung von geruchsarmen und lagerstabilen monomerhaltigen Polyisocyanuraten auf Basis von Isophorondiisocyanat hätte vor allem einen Verzicht auf die Entmonomerisierung zur Voraussetzung. Überraschend wurde gefunden, dass dieser Schritt tatsächlich eingespart und zudem der Einsatz von ggf. qualitätsmindernden Inhibitoren vermieden werden kann, wenn die Trimerisierung von IPDI in Gegenwart von speziellen Katalysatoren durchgeführt wird.

Gegenstand der Erfindung ist deshalb ein Verfahren zur Herstellung von geruchsarmen und lagerstabilen monomerhaltigen Polyisocyanuraten aus Isophorondiisocyanat, dadurch gekennzeichnet, dass die partielle Trimerisierung innerhalb von 3 Minuten bis 3 Stunden in Gegenwart von 0,05 - 2 Gew.-%, basierend auf dem Gewicht des Diisocyanats, eines Katalysators der allgemeinen Formel

[R-NX₃]^{m⊕} mY^{⊖}

in der Y⁻ für ein Carbonsäureanion mit 4 - 8 C-Atomen und R für eine β-Hydroxyalkylgruppe mit 2 - 6 C-Atomen steht und X eine Alkylengruppe mit 2 - 3 C-Atomen darstellt, wobei die drei Reste X über ein gemeinsames, ggf. partiell β-hydroxyalkyliertes Stickstoffsatom mit dem quarternären Stickstoff einen tricyclischen Ring bilden, der ggf. über eine zum Stickstoff α-, β- oder γ-ständige OH-Gruppe verfügt, bei einer Temperatur von 0 - 160 °C, vorzugsweise 40 - 120 °C, besonders bevorzugt 55 - 95 °C erfolgt, und m eine Zahl von 1,0 bis 2,0 bedeutet. Dabei kann auf eine Monomerabtrennung und chemische Deaktivierung des Trimeriserungskatalysators verzichtet werden.

Grundsätzlich können zur Trimerisierung geeignete Isocyanate nach verschiedenartigen Verfahren hergestellt werden (Annalen der Chemie 562 (1949), S. 75ff.). Technisch insbesondere bewährt hat sich die Herstellung durch Phosgenierung von organischen Polyaminen zu den entsprechenden Polycarbaminsäurechloriden und deren thermische Spaltung in organische Polyisocyanate und Chlorwasserstoff. Alternativ können organische Polyisocyanate auch ohne die Verwendung von Phosgen, d. h. nach phosgenfreien Verfahren, hergestellt werden. Nach Angaben der EP-A-126 299 (USP 4,596,678), EP-A-126 300 (USP 4,596,679) und EP-A-355443 (USP 5,087,739) beispielsweise können (cyclo)aliphatische Diisocyanate wie 1-Isocyanato-3-isocyanatomethyl-3,5,5-trimethylcyclohexan (Isophorondiisocyanat bzw. IPDI) zugänglich gemacht werden durch Umsetzung der zugrundeliegenden (cyclo)aliphatischen Diamine mit Harnstoff und Alkoholen zu (cyclo)aliphatischen Biscarbaminsäureestern und deren thermische Spaltung in die entsprechenden Diisocyanate und Alkohole.

Für das erfindungsgemäße Verfahren zur Herstellung von von geruchsarmen und lagerstabilen monomerhaltigen Polyisocyanuraten auf Basis von Isophorondiisocyanat ist es unwesentlich, über welchen Syntheseweg das eingesetzte IPDI hergestellt wurde. Es sei jedoch darauf hingewiesen, dass die zur Erreichung eines wunschgemäßen NCO-Gehaltes notwendige Katalysatormenge auch von der Qualität des Rohstoffes abhängt. Erfahrungsgemäß macht ein ansteigender Gehalt des IPDI an hydrolysierbaren Chlorverbindungen eine Erhöhung der Katalysatormenge erforderlich. Anscheinend übt das hydrolysierbare Chlor tendenziell einen inhibitiven Effekt auf den Kontakt aus.

Die erfindungsgemäße Herstellung der geruchsarmen und lagerstabilen monomerhaltigen Polyisocyanuraten auf Basis von Isophorondiisocyanat durch partielle Trimerisierung kann kontinuierlich (Rohrreaktor oder Kesselkaskade) erfolgen oder auch batchweise durchgeführt werden. Der Katalysator wird in geringer Konzentration zwischen 0,05 und 2 Gew.-% eingesetzt. Die exakte Menge läßt sich leicht experimentell ermitteln und ist abhängig vom Katalysator, vom Umsatzziel, von der Qualität des eingesetzten IPDI sowie der Verfahrensweise.

Die partielle Trimerisierung lässt sich innerhalb von 3 Minuten bis 3 Stunden durchführen. Das Produkt enthält monomeres IPDI, trimeres IPDI-Isocyanurat und höhere Oligomere mit Isocyanuratstruktur. Als Nebenkomponente können in geringer Menge ggf. auch Verbindungen mit Uretdionstruktur gefunden werden. Verbindungen dieser Art sind in der Literatur beschrieben.

Erfindungsgemäß wird der Katalysator in einer Menge von 0,05 - 2 Gew.-%, basierend auf dem Gewicht des eingesetzten Isophorondiisocyanats, eingesetzt.

Der Katalysator lässt sich leicht durch Reaktion der drei Grundbausteine - tricyclisches Diamin, Carbonsäure und Oxiran - erhalten. Die Herstellung kann ggf. in Gegenwart eines Lösemittels erfolgen. Üblicherweise kommen niedermolekulare Alkohole wie Methanol oder Ethylenglykol zum Einsatz. Als tricyclisches Diamin kommt z. B. Diazabicyclo[2.2.2]octan in Frage. Geeignete Carbonsäuren sind z. B. Essigsäure, Hexansäure oder 2-Ethylhexansäure. Mögliche Optionen für die Oxirankomponente sind z. B. Propylenoxid, Butylenoxid oder 1,2-Epoxyhexan. Die Molverhältnisse der drei Grundbausteine zur Herstellung der erfindungsgemäß eingesetzten Katalysatoren sind variabel. Je nach Verhältnis der Grundbausteine werden Katalysatoren mit mindestens einem quaternären Stickstoffatom im Molekül erhalten. Wie die Beispiele zeigen, werden auch solche Katalysatoren eingesetzt, die teilweise über zwei quaternäre Stickstoffatome verfügen.

Das erfindungsgemäße Verfahren wird bei Temperaturen zwischen 0 °C und 160 °C, vorzugweise zwischen 40 °C und 120 °C, und besonders bevorzugt zwischen 55 und 95 °C durchgeführt.

Erfindungsgemäß wird die partielle Trimerisierung des Isophorondiisocyanats entweder batchweise oder kontinuierlich durchgeführt. Das Batchverfahren wird bevorzugt.

Beim Batchverfahren wird in einem Rührreaktor gearbeitet. Dabei wird das Isophorondiisocyanat vorgelegt. Der Katalysator wird zudosiert, nachdem das IPDI die zur Reaktion notwendige Temperatur von 0 - 140 °C, vorzugsweise von 55 - 90 °C, besonders bevorzugt von 65 - 75 °C, erreicht hat. Die Trimerisierung ist exotherm. Der Katalysator wird zunächst in einer solchen Menge zudosiert, dass eine deutlicher Anstieg der Temperatur der Reaktionsmischung um 5 - 15 °C erkennbar wird. Da der Katalysator im Zuge der Reaktion deaktiviert wird, sinkt die Temperatur der Reaktionsmischung im Verlauf der Umsetzung wieder ab und eine erneute Katalysatordosierung kann erfolgen. Dieser Vorgang wird wiederholt bis der gewünschte Umsatz erreicht ist. Die Abfolge von Katalysatordeaktivierung und Reinitiierung der Trimerisierung durch portionsweise Katalysatornachdosierung erlaubt dabei jederzeit eine optimale Prozesskontrolle sowohl in bezug auf den Umsatz als auch hinsichtlich des Temperaturprofils der Reaktion.

Der Katalysator kann in reiner Form eingesetzt werden. Zur exakteren Dosierung und optimalen Durchmischung des Katalysators ist es jedoch vorteilhaft, den Katalysator in einem geeignetem Lösemittel zu lösen. Geeignet sind prinzipiell solche Lösemittel, in denen der Katalysator eine gute Löslichkeit hat, z. B. Wasser, niedermolekulare Alkohole wie Methanol oder Ethylenglykol oder niedermolekulare organische Säuren wie beispielsweise Essigsäure oder Hexansäure.

Die kontinuierlich Trimerisierung kann in einer Kesselkaskade durchgeführt werden. Denkbar ist auch eine Kombination aus Kesselkaskade und Rohrreaktor.

Zur Beschränkung der auf den gewünschten Umsatz bezogenen notwendigen Katalysatormenge sollte das Temperaturprofil des erfindungsgemäßen Verfahrens so eingerichtet werden, dass die Reaktionslösung eine Temperatur von 95 °C möglichst nicht überschreitet.

Die erfindungsgemäß hergestellten geruchsarmen und lagerstabilen monomerhaltigen Polyisocyanuraten auf Basis von Isophorondiisocyanat stellen nützliche Zwischenprodukte für Polyurethanbeschichtungen, Polyurethan- und Polyharnstoff-Formteile wie sie beispielsweise im Rahmen des RIM-Verfahrens ("Reaction Injection Moulding") produziert werden, für Polyurethan-Spritzapplikationen oder auch für Polyurethan-basierenden Automobilfensterverguß dar. Dabei können sie auch in mit Blockierungsmitteln blockierter Form zum Einsatz gelangen. Geeignete Blockierungsmittel sind dabei beispielsweise Lactame wie ε-Caprolactam, Oxime wie Methylethylketoxim oder Butanonoxim, Triazole wie 1H-1,2,4-Triazol, leicht enolisierbare Verbindungen wie Acetessigester oder Acetylaceton oder auch Malonsäurederivate wie Malonsäurediester.

### Beispiele

### Katalysatorherstellung

### Katalysator 1

70 Gew.-% einer Mischung aus Butylenoxid, 2-Ethylhexansäure und Diazabicyclo[2.2.2]octan (Molverhältnis 1:1:1) wurden in Gegenwart von 30 Gew.-% Ethylenglykol 3 d bei Raumtemperatur gerührt.

### Katalysator 2

70 Gew.-% einer Mischung aus Propylenoxid, 2-Ethylhexansäure und Diazabicyclo[2.2.2]octan (Molverhältnis 1:1:1) wurden in Gegenwart von 30 Gew.-% Ethylenglykol 3 d bei Raumtemperatur gerührt.

### Katalysator3

60 Gew.-% einer Mischung aus Propylenoxid, 2-Ethylhexansäure und Diazabicyclo[2.2.2]octan (Molverhältnis 1,25:1,25:1,12) wurden in Gegenwart von 40 Gew.-% Ethylenglykol 3 d bei Raumtemperatur gerührt.

### Katalysator 4

80 Gew.-% einer Mischung aus Propylenoxid, 2-Ethylhexansäure und Diazabicyclo[2.2.2]octan (Molverhältnis 1,4:1,4:1) wurden in Gegenwart von 20 Gew.-% Methanol 3 d bei Raumtemperatur gerührt.

### Trimerisierungsexperimente: Beispiele 1 - 4 und Vergleichsbeispiele 1 - 6

Die Umsetzungen erfolgten grundsätzlich unter einer N₂-Atmosphäre.

### Beispiele

### A.1. Trimerisierung von IPDI mit Katalysator 1

800 g IPDI wurden bei 70 °C portionsweise mit 2,0 g (0,25 Gew.-%) Katalysator 1 versetzt. Die Temperatur der mechanisch gerührten Reaktionsmischung stieg auf einen Maximalwert von 87 °C an und fiel im Anschluß wieder langsam ab. Es wurde nun in kleinen Portionen weiterer Katalysator zugesetzt, so dass die Temperatur der Reaktionslösung sich im Bereich von 81 - 90 °C bewegte. Nach Zudosierung von insgesamt 3,9 g (0,49 Gew.-%) Katalysator 1 hatte die Reaktionsmischung einen NCO-Gehalt von unter 29 Gew.-% erreicht. Man ließ auf Raumtemperatur abkühlen. Der NCO-Gehalt des geruchsarmen Reaktionsproduktes betrug 28,2 % und blieb auch bei Lagerung (7 d, 20 - 30 °C) stabil.

### A.2. Trimerisierung von IPDI mit Katalysator 2

800 g IPDI wurden bei 70°C portionsweise mit 2,0 g (0,25 Gew.-%) Katalysator 2 versetzt. Die Temperatur der mechanisch gerührten Reaktionsmischung stieg auf einen Maximalwert von 89 °C an und fiel im Anschluß wieder langsam ab. Es wurde nun in kleinen Portionen weiterer Katalysator zugesetzt, so dass die Temperatur der Reaktionslösung sich im Bereich von 78 - 90 °C bewegte. Nach Zudosierung von insgesamt 3,7 g (0,46 Gew.-%) Katalysator 2 hatte die Reaktionsmischung einen NCO-Gehalt von unter 29 Gew.-% erreicht. Man ließ auf Raumtemperatur abkühlen. Der NCO-Gehalt des geruchsarmen Reaktionsproduktes betrug 28,4 % und blieb auch bei Lagerung (7 d, 20 - 30 °C) stabil.

### B.3. Trimerisierung von IPDI mit Katalysator 3

50 kg IPDI wurden bei 70 °C portionsweise mit 102 g g (0,20 Gew.-%) Katalysator 3 versetzt. Die Temperatur der mechanisch gerührten Reaktionsmischung stieg auf einen Maximalwert von 85 °C an und fiel im Anschluß wieder langsam ab. Es wurde nun in kleinen Portionen weiterer Katalysator zugesetzt, so dass die Temperatur der Reaktionslösung sich im Bereich von 83 - 92 °C bewegte. Nach Zudosierung von insgesamt 223 g (0,45 Gew.-%) Katalysator 3 hatte die Reaktionsmischung einen NCO-Gehalt von unter 29 Gew.-% erreicht. Man ließ auf Raumtemperatur abkühlen. Der NCO-Gehalt des geruchsarmen Reaktionsproduktes betrug 28,1 % und blieb auch bei Lagerung (7 d, 20 - 30 °C) stabil.

### B.4. Trimerisierung von IPDI mit Katalysator 4

50 kg IPDI wurden bei 70 °C portionsweise mit 110 g (0,22 Gew.-%) Katalysator 4 versetzt. Die Temperatur der mechanisch gerührten Reaktionsmischung stieg auf einen Maximalwert von 86 °C an und fiel im Anschluß wieder langsam ab. Es wurde nun in kleinen Portionen weiterer Katalysator zugesetzt, so dass die Temperatur der Reaktionslösung sich im Bereich von 82 - 94 °C bewegte. Nach Zudosierung von insgesamt 220 g (0,44 Gew.-%) Katalysator 4 hatte die Reaktionsmischung einen NCO-Gehalt von unter 29 Gew.-% erreicht. Man ließ auf Raumtemperatur abkühlen. Der NCO-Gehalt des geruchsarmen Reaktionsproduktes betrug 28,4 % und blieb auch bei Lagerung (7 d, 20 - 30 °C) stabil.

### Vergleichsbeisspiele

### B.5. Trimersierung von IPDI mit Dabco TMR^{R}

1500 g IPDI wurden bei 80 °C mit 3,75 g (0,25 Gew.-%) Dabco TMR^{R} (N-(2-Hydroxypropyl)-N,N,N-trimethylammonium-2-ethylhexanoat, ca. 75%ig in Diethylenglykol) versetzt. Die Temperatur der mechanisch gerührten Reaktionsmischung stieg aufgrund des stark exothermen Verlaufs der Umsetzung innerhalb von ca. 3 min auf einen Spitzenwert von 136 °C an. Man ließ auf Raumtemperatur abkühlen. Der NCO-Gehalt des stark aminisch riechenden Reaktionsproduktes betrug 28,9 % und blieb auch bei Lagerung (7 d, 20 - 30 °C) stabil.

Zur Beseitigung des Geruchsproblems wurde nicht umgesetztes IPDI durch Kurzwegverdampfung vom Polyisocyanat abgetrennt. Nach Verdünnung des monomerbefreiten Harzes mit frischem IPDI auf einen NCO-Gehalt von 29,6 % wurde ein geruchsarmes monomerhaltiges IPDI-Trimer erhalten.

### B.6. Trimerisierung von IPDI mit Dabco TMR^{R}-2

1500 g IPDI wurden bei 80 °C mit 3,75 g (0,25 Gew.-%) Dabco TMR^{R}-2 (N-(2-Hydroxypropyl)-N,N,N-trimethylammoniumformat, ca. 75%ig in Diethylenglykol) versetzt. Die Temperatur der mechanisch gerührten Reaktionsmischung stieg aufgrund des stark exothermen Verlaufs der Umsetzung innerhalb von ca. 3 min auf einen Spitzenwert von 139 °C an. Man ließ auf Raumtemperatur abkühlen. Der NCO-Gehalt des stark aminisch riechenden Reaktionsproduktes betrug 28,2 % und blieb auch bei Lagerung (7 d, 20 - 30 °C) stabil.

Zur Beseitigung des Geruchsproblems wurde nicht umgesetztes IPDI durch Kurzwegverdampfung vom Polyisocyanat abgetrennt. Nach Verdünnung des monomerbefreiten Harzes mit frischem IPDI auf einen NCO-Gehalt von 29,6 % wurde ein geruchsarmes monomerhaltiges IPDI-Trimer erhalten.

### B.7. Trimerisierung von IPDI mit N-(2-Hydroxypropyl)-N,N,N-trimethylammoniumhydroxid

1500 g IPDI wurden bei 80 °C mit 3,75 g (0,25 Gew.-%) N-(2-Hydroxypropyl)-N,N,N-trimethylammoniumhydroxid (ca. 75%ig in Diethylenglykol) versetzt. Die Temperatur der mechanisch gerührten Reaktionsmischung stieg aufgrund des stark exothermen Verlaufs der Umsetzung innerhalb von ca. 3 min auf einen Spitzenwert von 143 °C an. Man ließ auf Raumtemperatur abkühlen. Der NCO-Gehalt des stark aminisch riechenden Reaktionsproduktes betrug 27,6 % und blieb auch bei Lagerung (7 d, 20 - 30 °C) stabil.

Zur Beseitigung des Geruchsproblems wurde nicht umgesetztes IPDI durch Kurzwegverdampfung vom Polyisocyanat abgetrennt. Nach Verdünnung des monomerbefreiten Harzes mit frischem IPDI auf einen NCO-Gehalt von 29,6 % wurde ein geruchsarmes monomerhaltiges IPDI-Trimer erhalten.

### B.8. Trimerisierung von IPDI mit Hexamethyldisilazan (HMDS)

1600 g IPDI wurden bei 100 °C mit 1,6 g (1 Gew.-%, 0,1 mol) HMDS versetzt. Nachdem nach 30 min kein Umsatz beobachtet werden konnte, wurde die Temperatur der mechanisch gerührten Reaktionsmischung auf 120 °C erhöht. Auch unter diesen Bedingungen ließ sich kein nennenswerter Umsatz erzielen. Man ließ auf 50 °C abkühlen und deaktivierte den Katalysator durch Zusatz von 0,9 g (0,05 mol) Wasser. Die Reaktionslösung wies einen NCO-Gehalt von 37,2 % auf und sonderte einen aminartigen Geruch ab. Aufgrund des geringen Umsatzes wurde auf eine Beseitigung des Geruchsproblems durch Kurzwegverdampfung und anschließende Verdünnung des monomerbefreiten Harzes mit frischem IPDI verzichtet.

### B.9. Trimerisierung von IPDI mit Dabco TMR^{R}

1500 g IPDI wurden bei 70 °C mit 2,2 g (0,15 Gew.-%) Dabco TMR^{R} (N-(2-Hydroxypropyl)-N,N,N-trimethylammonium-2-ethylhexanoat, ca. 75%ig in Diethylenglykol) versetzt. Die Temperatur der mechanisch gerührten Reaktionsmischung stieg auf einen Spitzenwert von 75 °C an. Die Temperatur der Reaktionsmischung ließ sich ohne weitere Katalysatordosierung auf einem Niveau von 70 - 75 °C halten. Nachdem die Reaktionsmischung einen NCO-Gehalt von 29 Gew.-% unterschritten hatte, ließ man auf Raumtemperatur abkühlen. Der NCO-Gehalt des stark aminisch riechenden Reaktionsproduktes betrug 28,3 %. Das Reaktionsprodukt verhielt sich nicht lagerstabil. Bei Lagerung (7 d, 20 - 30 °C) fiel der NCO-Gehalt als Funktion der Zeit ab.

Zur Beseitigung des Geruchsproblems wurde nicht umgesetztes IPDI durch Kurzwegverdampfung vom Polyisocyanat abgetrennt. Nach Verdünnung des monomerbefreiten Harzes mit frischem IPDI auf einen NCO-Gehalt von 29,6 % wurde ein geruchsarmes monomers haltiges IPDI-Trimer erhalten.

### B.10. Trimerisierung von IPDI mit Dabco TMR^{R}-2

1500 g IPDI wurden bei 70 °C mit 2,4 g (0,16 Gew.-%) Dabco TMR^{R}-2 (N-(2-Hydroxypropyl)-N,N,N-trimethylammoniumformat, ca. 75%ig in Diethylenglykol) versetzt. Die Temperatur der mechanisch gerührten Reaktionsmischung stieg auf einen Spitzenwert von 76 °C an. Die Temperatur der Reaktionsmischung ließ sich ohne weitere Katalysatordosierung auf einem Niveau von 70 - 75 °C halten. Nachdem die Reaktionsmischung einen NCO-Gehalt von 29 Gew.-% unterschritten hatte, ließ man auf Raumtemperatur abkühlen. Der NCO-Gehalt des stark aminisch riechenden Reaktionsproduktes betrug 28,5 %. Das Reaktionsprodukt verhielt sich nicht lagerstabil. Bei Lagerung (7 d, 20 - 30 °C) fiel der NCO-Gehalt als Funktion der Zeit ab.

Zur Beseitigung des Geruchsproblems wurde nicht umgesetztes IPDI durch Kurzwegverdampfung vom Polyisocyanat abgetrennt. Nach Verdünnung des monomerbefreiten Harzes mit frischem IPDI auf einen NCO-Gehalt von 29,6 % wurde ein geruchsarmes monomerhaltiges IPDI-Trimer erhalten.

**Tabelle 1 Trimerisierungen von IPDI (Beispiele B.1.-B.4. u. Vergleichsbeispiele B.5.-B.10.)**

| Versuch | Kategorie | Katalysator | Kat menge [Gew.-%] | NCO- Gehalt [Gew.-%] | Bermerkungen |
|---|---|---|---|---|---|
| B.1. | Beispiel | Katalysator 1 | 0,49 | 28,2 | lagerstabil, |
| | | | | | geruchsarm |
| B.2. | Beispiel | Katalysator 2 | 0,46 | 28,4 | lagerstabil, |
| | | | | | geruchsarm |
| B.3. | Beispiel | Katalysator 3 | 0,45 | 28,1 | lagerstabil, |
| | | | | | geruchsarm |
| B.4. | Beispiel | Katalysator 4 | 0,44 | 28,4 | lagerstabil, |
| | | | | | geruchsarm |
| B.5. | Vergleichsbsp. | Dabco TMR^{R} | 0,25 | 28,9 | lagerstabil, deutliche |
| | | | | | Geruchsentwicklung |
| B.6. | Vergleichsbsp. | Dabco TMR^{R}-2 | 0,25 | 28,2 | lagerstabil, deutliche |
| | | | | | Geruchsentwicklung |
| B.7. | Vergleichsbsp. | N-(2-Hydroxypropyl)- | 0,25 | 27,6 | lagerstabil, deutliche |
| | | N,N,N-trimethyl-ammoniumhydroxid | | | Geruchsentwicklung |
| B.8. | Vergleichsbsp. | Hexamethyldisilazan | 1,0 | 37,2 | lagerstabil, deutliche |
| | | | | | Geruchsentwicklung |
| B.9. | Vergleichsbsp. | Dabco TMR^{R} | 0,15 | 28,3 | nicht lagerstabil, deutliche |
| | | | | | Geruchsentwicklung |
| B.10 | Vergleichsbsp. | Dabco TMR^{R}-2 | 0,16 | 28,5 | nicht lagerstabil, deutliche |
| | | | | | Geruchsentwicklung |

## Patentansprüche

1. Verfahren zur Herstellung von geruchsarmen und lagerstabilen monomerhaltigen Polyisocyanuraten aus Isophorondiisocyanat, **dadurch gekennzeichnet, dass** die partielle Trimerisierung innerhalb von 3 Minuten bis 3 Stunden in Gegenwart von 0,05 - 2 Gew.-%, basierend auf dem Gewicht des Diisocyanats, eines Katalysators der allgemeinen Formel
[R-NX₃]^{m⊕} mY^{⊖}
in der Y⁻ für ein Carbonsäureanion mit 4 - 8 C-Atomen und R für eine β-Hydroxyalkylgruppe mit 2 - 6 C-Atomen steht und X eine Alkylengruppe mit 2 - 3 C-Atomen darstellt, wobei die drei Reste X über ein gemeinsames, ggf. partiell β-hydroxyalkyliertes Stickstoffatom mit dem quarternären Stickstoff einen tricyclischen Ring bilden, der ggf. über eine zum Stickstoff α- oder β- oder γ-ständige OH-Gruppe verfügt, bei einer Temperatur von 0 - 160 °C erfolgt, und m eine Zahl von 1,0 bis 2,0 bedeutet.

2. Verfahren gemäß Anspruch 1,
**dadurch gekennzeichnet,**
**dass** ein Isophorondiisocyanat eingesetzt wird, das nach einem Phosgenverfahren oder einem phosgenfreien Prozeß hergestellt wurde.

3. Verfahren nach mindestens einem der Ansprüche 1 bis 2,
**dadurch gekennzeichnet,**
**dass** das lagerstabile monomerhaltige Polyisocyanurate auf Basis von Isophorondiisocyanat einen NCO-Gehalt von 22 - 34 Gew.-% aufweist.

4. Verfahren nach mindestens einem der Ansprüche 1 bis 3,
**dadurch gekennzeichnet,**
**dass** die Umsetzung bei Temperaturen von 40 bis 120 °C erfolgt.

5. Verfahren nach mindestens einem der Ansprüche 1 bis 4,
**dadurch gekennzeichnet,**
**dass** die Umsetzung bei Temperaturen von 55 bis 95 °C erfolgt.

6. Verfahren nach mindestens einem der Ansprüche 1 bis 5,
**dadurch gekennzeichnet,**
**dass** auf eine Monomerabtrennung verzichtet wird.

7. Verfahren nach mindestens einem der Ansprüche 1 bis 6,
**dadurch gekennzeichnet,**
**dass** auf eine chemische Deaktivierung des Trimeriserungskatalysators verzichtet wird.

8. Verfahren nach mindestens einem der Ansprüche 1 bis 7,
**dadurch gekennzeichnet,**
**dass** es batchweise durchgeführt wird.

9. Verfahren nach mindestens einem der Ansprüche 1 bis 8,
**dadurch gekennzeichnet,**
**dass** es kontinuierlich durchgeführt wird.

10. Verfahren nach mindestens einem der Ansprüche 1 bis 9,
**dadurch gekennzeichnet,**
**dass** es kontinuierlich in einem Rohrreaktor durchgeführt wird.

11. Verfahren nach mindestens einem der Ansprüche 1 bis 10,
**dadurch gekennzeichnet,**
**dass** es kontinuierlich in einer Kesselkaskade durchgeführt wird.

12. Verfahren nach mindestens einem der Ansprüche 1 bis 11,
**dadurch gekennzeichnet,**
**dass** es kontinuierlich in einer Kesselkaskade und einem Rohrreaktor durchgeführt wird.

## Claims

1. Process for preparing low-odour storage-stable monomer-containing polyisocyanurates from isophorone diisocyanate, **characterized in that** the partial trimerization is conducted over from 3 minutes to 3 hours in the presence of 0.05-2% by weight, based on the weight of the diisocyanate, of a catalyst of the general formula
[R-NX₃]^{m⊕} mY^{⊖}
where Y- is a carboxylic acid anion of 4-8 carbons and R is a β-hydroxyalkyl group of 2-6 carbons and X is an alkylene group of 2-3 carbons, the three radicals X forming a tricyclic ring with the quaternary nitrogen by way of a common nitrogen atom, which may be partly β-hydroxyalkylated, said tricyclic ring possibly having an OH group positioned α, β or γ to the nitrogen, at a temperature of 0-160°C, and m is a number from 1.0 to 2.0.

2. Process according to Claim 1, **characterized in that** an isophorone diisocyanate is used that has been prepared by a phosgene process or by a phosgene-free process.

3. Process according to at least one of Claims 1 to 2, **characterized in that** the storage-stable monomer-containing polyisocyanurate based on isophorone diisocyanate has an NCO content of 22-34% by weight.

4. Process according to at least one of Claims 1 to 3, **characterized in that** the reaction takes place at temperatures from 40 to,120°C.

5. Process according to at least one of Claims 1 to 4, **characterized in that** the reaction takes place at temperatures from 55 to 95°C.

6. Process according to at least one of Claims 1 to 5, **characterized in that** no separation of monomer is performed.

7. Process according to at least one of Claims 1 to 6, **characterized in that** no chemical deactivation of the trimerization catalyst is performed.

8. Process according to at least one of Claims 1 to 7, **characterized in that** it is carried out batchwise.

9. Process according to at least one of Claims 1 to 8, **characterized in that** it is carried out continuously.

10. Process according to at least one of Claims 1 to 9, **characterized in that** it is carried out continuously in a tube reactor.

11. Process according to at least one of Claims 1 to 10, **characterized in that** it is carried out continuously in a tank cascade.

12. Process according to at least one of Claims 1 to 11, **characterized in that** it is carried out continuously in a tank cascade and a tube reactor.

## Revendications

1. Procédé en vue de la fabrication de polyisocyanurates contenant des monomères, avec une faible odeur et stables au stockage, faits à partir de diisocyanate d'isophorone, **caractérisé en ce que** la trimérisation partielle se fait à une température de 0 - 160 °C en l'espace de 3 minutes à 3 heures, en présence de 0,05 - 2 % en poids, sur la base du poids du diisocyanate, d'un catalyseur de la formule générale
[R-NX₃]^{m⊕} mY^{⊖}
dans laquelle Y⁻ représente un anion d'acide carboxylique ayant 4 - 8 atomes de C et R désigne un groupement β-hydroxylalkyle ayant 2 - 6 atomes de C et X représentant un groupement alcylène ayant 2 - 3 atomes de C, les trois radicaux X formant par l'intermédiaire d'un atome d'azote commun, le cas échéant, partiellement β-hydroxyalkylé avec l'azote quaternaire, un cycle tricylclique qui dispose, le cas échéant, d'un groupement OH en position α, β ou γ par rapport à l'azote et **en ce que** m désigne un nombre de 1,0 à 2, 0.

2. Procédé selon la revendication 1, **caractérisé en ce que** l'on utilise un diisocyanate d'isophorone, qui a été fabriqué à l'aide d'un procédé au phosgène ou à l'aide d'un processus exempt de phosgène.

3. Procédé selon au moins une des revendications 1 à 2, **caractérisé en ce que** le polyisocyanurate contenant des monomères, stable au stockage à base de diisocyanate d'isophorone présente une teneur en NCO de 22 - 34 % en poids.

4. Procédé selon au moins une des revendications 1 à 3, **caractérisé en ce que** la réaction se fait à des températures de 40 à 120 °C.

5. Procédé selon au moins une des revendications précédentes 1 à 4, **caractérisé en ce que** la réaction se fait à des températures de 55 à 95 °C.

6. Procédé selon au moins une des revendications 1 à 5, **caractérisé en ce que** l'on renonce à une séparation des monomères.

7. Procédé selon au moins une des revendications 1 à 6, **caractérisé en ce que** l'on renonce à une désactivation chimique du catalyseur de trimérisation.

8. Procédé selon au moins une des revendications 1 à 7, **caractérisé en ce qu'**il est effectué en mode discontinu.

9. Procédé selon au moins une des revendications 1 à 8, **caractérisé en ce qu'**il est effectué en mode continu.

10. Procédé selon au moins une des revendications 1 à 9, **caractérisé en ce qu'**il est effectué en mode continu dans un réacteur tubulaire.

11. Procédé selon au moins une des revendications 1 à 10, **caractérisé en ce qu'**il est effectué dans une cascade de cuves.

12. Procédé selon au moins une des revendications 1 à 11, **caractérisé en ce qu'**il est effectué en mode continu dans une cascade de cuves et dans un réacteur tubulaire.
